# EUROPEAN PATENT SPECIFICATION

(11) **EP 3 240 527 B1**
(45) Date of publication and mention of the grant of the patent: **25.09.2019**
(21) Application number: 14845013.3
(22) Date of filing: 31.12.2014
(51) Int. Cl.: A61K 9/00, A61K 9/16, A61K 36/752, A61K 36/899, A61K 33/00, A61K 36/53, A61K 36/54

(54) **ESSENTIAL OIL LOADED MUCOADHESIVE NANOCOMPOSITE DELIVERY SYSTEM FOR GASTROINTESTINAL SYSTEM**
SYSTEM ZUR AUSGABE VON MUCOADHÄSIVEM, MIT ÄTHERISCHEM ÖL BELADENEM NANOKOMPOSIT FÜR MAGEN-DARM-SYSTEM
SYSTÈME D'ADMINISTRATION DE NANOCOMPOSITE MUCO-ADHÉSIF CHARGÉ D'HUILE ESSENTIELLE POUR LE SYSTÈME GASTRO-INTESTINAL

(43) Date of publication of application: 08.11.2017
(73) Proprietor: Tihminlioglu, Funda, 35430 Urla/Izmir (TR); Izmir Yuksek Teknoloji Enstitusu, 35430 Urla/Izmir (TR); Altiok, Duygu, 34303 Kucukcekmece/Istanbul (TR); Gunes, Suna Seda, 35430 Urla/Izmir (TR); Izmir Teknoloji Gelistirme Bolgesi A.S., 35340 Urla/Izmir (TR)
(72) Inventor: ALTIOK, Duygu, 34303 Kucukcekmece/Istanbul (TR); TIHMINLIOGLU, Funda, 35430 Urla/Izmir (TR); GUNES, Suna Seda, 35330 Balçova-Izmir (TR)
(86) International application number: PCT/TR2014/000542
(87) International publication number: WO 2016/108774

(56) References cited:
- EP-A1- 1 178 104
- EP-A1- 1 985 585
- MEHDI ABDOLLAHI ET AL: "A novel active bionanocomposite film incorporating rosemary essential oil and nanoclay into chitosan", JOURNAL OF FOOD ENGINEERING, BARKING, ESSEX, GB, vol. 111, no. 2, 7 February 2012 (2012-02-07), pages 343-350, XP028476668, ISSN: 0260-8774, DOI: 10.1016/J.JFOODENG.2012.02.012 [retrieved on 2012-02-16]
- YUAN Q ET AL: "Controlled and extended drug release behavior of chitosan-based nanoparticle carrier", ACTA BIOMATERIALIA, ELSEVIER, AMSTERDAM, NL, vol. 6, no. 3, 1 March 2010 (2010-03-01), pages 1140-1148, XP026879159, ISSN: 1742-7061 [retrieved on 2009-08-21]

## Description

### Technical Field

This invention relates to a local treatment of infection caused by *Helicobacter pylori* (*H.pylori*) and/or improve the drug release profiles of encapsulated essential oils in chitosan/nanoclay microspheres which are localized in gastrointestinal system. The specific essential oils are cinnamon, thyme, lemongrass, lemon and clove which have antimicrobial activity against *H*. *pylori.*

### Background of the Invention (Prior art)

*H.pylori* is a Gram-negative bacterium which has high prevalence in the world, plays role in cronic gastritis, peptic ulcer and is associated with mucosal related lenfoid tissue lymphoma (MALT). Successful treatment of *H*. *pylori* infection requires combination therapy, consisting of one or two antibiotics, an acid inhibitor and/or a bismuth component. However, none of these drugs is effective enough to eradicate *H*. *pylori* in monotherapy and, such a combination of treatments does not always offer a complete cure and undesirable side effects are often observed. *In vitro, H. pylori* is susceptible to several antibiotics, but *in vivo* only a small number of them can be used successfully for eradication of *H. pylori.* The increased prevalence of antibiotic resistance in *H. pylori* is a serious problem because it is the major cause of treatment failure. However the major challanges in the current treatment of *H.pylori* were side-effects of antibiotics, poor diffusion of antibiotics to mucus layer, insufficiant residence time in the stomach and low stability and antibiotic resistance. Consequently alternative therapies including newer drugs and therapeutic approaches with enhanced action and reduced side effects are needed for better eradication of the bacteria. In regions of high clarithromycin resistance (>20%), alternative therapies should be recommended.

Specific essential oils obtained from different plant source show anti-Helicobacter activity *in vitro.* Cinnamon bark oil, thyme, clove, lemongrass and lemon oil have strong inhibitory effect on *Helicobacter pylori.* Essential oil (EO) is a volatile product of a plant secondary metabolism and can be extracted by steam distillation, supecritical fluid extraction. Cinnamon, clove, thyme, lemongrass and lemon oil have biological activities such as antioxidant, antimicrobial and anti-inflammatory activity and cinnamon oil has antiulserogenic activity. The major components of cinnamon, clove, thyme, lemongrass and lemon oil are cinnamaldehyde, eugenol, thymol, geraniol and nerol, lemonene are responsible for antimicrobial activity and have antitumor, antifungal and non-cytotoxic properties.

Chitosan is polycationic, biodegradable, biocompatible, mucoadhesive and the most abundant biopolymer type in the world after cellulose. It is a subitable drug carrier agent especially due to its mucoadhesive, biodegradable and wound healing properties. Moreover, it has analgesic, antitumoral, hemostatic, hypocholesterolemic, antimicrobial and antioxidant activities. Chitosan shows bacteriostatic property by interacting with negatively charged bacterial cell wall due to its polycationic nature. The interaction between the positively charged chitosan and the negatively charged bacterial cell wall leads to the leakage of intracellular components and thus the death of the organism. Chitosan based drug carrier systems are preferable for preventing gastric colonization of *H.pylori* due to its mucoadhesive and bacteriostatic properties.
Chitosan microspheres have been described as drug delivery systems previously. Many studies have been performed to eradicate *H. pylori* and these studies are listed in Table 1. The effects of parameters such as the drug/polymer ratio, stirring speed on drug entrapment efficiency, particle size, degree of swelling and drug release kinetics of antibiotic loaded microspheres such as amoxicillin, clarithromycin, tetracycline or metronidazole were investigated. However, related studies cannot provide that essential oil loaded chitosan microspheres for treatment of *H*. *pylori* with a controlled release profile.

**Table 1. Antibiotic release systems for H. pylori**

| **Polymer** | **System** | **Used Technique** | **Crosslinking Agent** | **Drug** | **Source** |
|---|---|---|---|---|---|
| Chitosan Polyacrylic Acid | Hydrogel | Spray drying | - | Amoxicillin | (Torre et al., 2003) |
| Chitosan | Microsphere | Emulsification | Glutaraldehyde | Amoxicillin | (Patel and Patel, 2007) |
| Chitosan | Microsphere | Emulsification | Glutaraldehyde | Clarithromycin | (Majithiya and Murthy, 2005) |
| Chitosan Polyacrylic Acid | Hydrogel | Spray drying | - | Amoxicillin | (Torre et al., 2005) |
| Chitosan | Microsphere | Ionic crosslinking/ Precipitation | - | Tetracycline | (Hejazi and Amiji, 2002) |
| Chitosan | Microsphere | Chemical crosslinking/ Precipitation | Glyoxal | Tetracycline | (Hejazi and Amiji, 2004) |
| Chitosan Polyethylene oxide | Hydrogel | Spray drying | Glyoxal | Amoxicillin metranidazole | (Patel and Amiji, 1996) |
| Chitosan Polyacrylic Acid | Hydrogel | Spray drying | - | Amoxicillin | (Torrado et al., 2004) |

Nanoclays are nano-sized natural materials of layered mineral silicates and depending on chemical composition and nanoparticle morphology, nanoclays are organized into several classes such as montmorillonite (MMT), bentonite, kaolinite, hectorite, and halloysite. Nanoclays improve mechanical, thermal and barrier properties of polymeric materials. Montmorillonite clay belongs the family of layered silicates (alumino-silicates) and has been commonly used in the research studies. Layered silicates have a 2:1 layered structure, in which one aluminum octahedral sheet is sandwiched between two silicon tetrahedral sheets (1). Polymer/clay nanocomposites are hybrid materials in which inorganic or organo-clay nanoparticles (layered silicate/nanoclay) are dispersed in a polymer matrix. The addition of the nanoclay platelets may improve the overall parmaceutical active ingredient (API) stability by providing a tortuous path that would slow down the API's diffusion to the body. Layered silicates with an efficient distribution in biopolymer matrix provide tortuous pathway (2) for molecules which diffuse through the composite, retard diffusion and provide a barrier by decreasing permeability. The decrease in permeabilities of chitosan/nanoclay nanocomposite could be explained by the good dispersion of clay platelet with large aspect ratios in the polymer matrix. This forces water vapor to follow a tortuous path through the polymer matrix surrounding the clay particles by increasing the effective path length for diffusion. Thus, the release of active ingredients is slowed down. This characteristic property could be used in the development of sustained drug release applications, prolonging the residence time of drug and controlled release.

The mostly used clay in polymer nanocomposites is montmorillonite (MMT) which is the main component of bentonite. MMT is a clay mineral providing controlled release and swelling property. Because the release of drugs in drug-intercalated layered materials is potentially controllable, these new materials have a great potential as a delivery host in the pharmaceutical field. Cationic montmorillonite, MMT, is a potent detoxifier and it can adsorb dietary toxins, bacterial and metabolic toxins. Calcium montmorillonite has also been used extensively in the treatment of pain, open wounds, colitis, diarrhea, hemorrhoids, stomach ulcers, intestinal problems, acne and anemia.

One embodiment of the prior art is U.S. Pat. No. 6,207,197 B1 and relates to prolong the pharmaceutical agents in the stomach of a mammal, provide local treatment of diseases of the stomach, or to improve the intestinal absorption of drugs. Related invention comprises an inner core containing a therapeutic agent (i.e. active ingredient or drug), insoluble polymer and an outer layer of a bioadhesive cationic polymer, also, it was recommended that this invention could be used as a kit for the treatment of *H.pylori* infection.

In one another embodiment is E.P. Publication. No. 0689842 A1 and relates to the use of an organic extract of Cinnamon to inhibit the growth of H. pylori and urease activity of H. pylori. Also E.P. Pat. 1178104 A1 describes a nutritional composition comprising a specific essential oil for prevention or treatment of infection by an helicobacter-like organism (HLO). However these related inventions does not include encapsulation of essential oils and controlled release mechanisms which provide more efficient eradication therapy by prolonging the retention time in stomach.

Another embodiment is US Pat. No. 2009/0232899 A1 and related with a drug carrier system binding a drug to the chitosan/silica nanocomposite. This invention also relates to a composite drug delivery system wherein chitosan is encapsulated with surface modified colloidal nanoparticles. This invention also relates to treatment of peptic ulser caused by *H. pylori* by delivering a nanopore composite of chitosan.

In one embodiment is WO 2013164652 A2 and related with the use of microspheres for binding *H. pylori* before and after adhesion to the gastric mucosa and/or mucosal layer. This invention provides a microsphere which comprises specific receptor for *H. pylori* binding and applied for removing *H. pylori* from stomach.

None of these cited references encomprises the encapsulation and gastroretentive controlled release of essential oils from mucoadhesive polymers for treatment of *H.pylori.* It is known that prolonged residence time in stomach, bioavailability and controlled release properties are also important as well as drug efficacy. The present invention relates to encapsulation of specific essential oil which is active against *H.pylori* and developmentment of a gastroretentive, controlled release composition that is retained in the stomach and releases the essential oil for an extended period of time.

### Summary of Invention

The claims disclose a chitosan-nanoclay nanocomposite controlled release system comprising essential oil and nanoclay, for use in the local treatment of infection caused by Helicobacter pylori. There are number of studies related with controlled release of antibiotics such as clarithromycin on *H. pylori* in prior art of invention. Furthermore, there are many embodiments that investigate the effectiveness of essential oils on *H.pylori.* However, as seen in the literature, there could not be found reported studies related with controlled release of essential oils (cinnamon, thyme, clove, lemongrass and lemon) from chitosan microspheres which are located at stomach and antimicrobial efficacy on *H.pylori.* This invention relates to encapsulation of essential oils in gastroretentive chitosan/nanoclay spheres which are localized in gastrointestinal system for local treatment of infection caused by *Helicobacter pylori* (*H.pylori*) and/or improve the drug release. The main advantages of this invention are:
- Decreasing the side and toxic effects of synthetic drugs by using biopolymers such as chitosan,
- Development of mucoadhesive gastroretentive system with controlled release of essential oil which has antimicrobial activity against *H.pylori.*
- Prevention adhesion of *H.pylori* to gastric mucosa with mucoadhesive chitosan and thus providing preventive therapy as well as eradication.
- Regeneration of damaged gastric tissue by this chitosan and essential oil containing nanocomposite.
- Enhancing gastric residence time and provide sustained release.

Therefore, the aim of this invention is to encapsulate essential oil in gastroretentive chitosan/nanoclay spheres which has antimicrobial activity against *H.pylori* and release in a controlled manner in the prior to art.

### Description of Figures

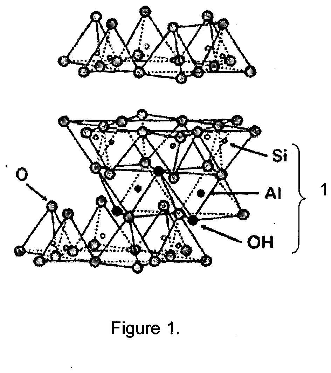

Figure 1; Molecular structure of montmorillonite clay

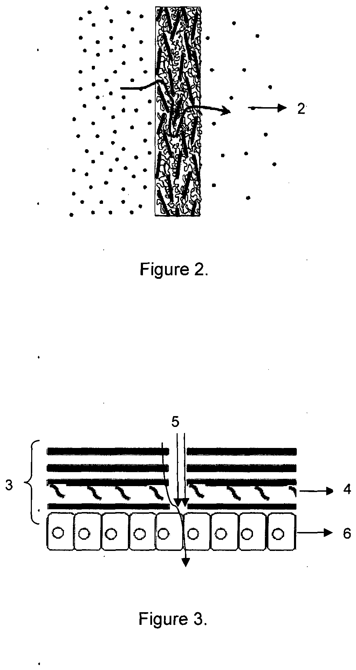

Figure 2; Dispersion of nanoclay in polymer matrix and controlled release mechanism with tortuous pathway
Figure 3; Interaction of chitosan nanocomposite with mucus layer and increasing permeability

### Description of references

1; 2:1 Layered structure of montmorillonite
2; Diffusion of molecules in a tortuous pathway
3; Mucus layer
4; *H.pylori*
5;lncreasing the passage of active agent through the mucus layer by chitosan nanocomposite
6; Epithelial cells

### Detailed Description of Invention

The related invention discloses a gastroretentive chitosan/nanoclay controlled release system comprising a therapeutically active amount of essential oil with improved stability. This invention is effective in prevention/removal of *H.pylori* adhesion to gastric cells.

Currently used treatment protocols for *H. pylori* eradication could not be achieved complete success because of the following reasons. Therefore, studies on alternative drugs and eradication protocols are being continued. Although many antibiotics show bacteriostatic and bactericidal effect *in vitro* at MIC level, a complete eradication could not be ensured. The main reasons are:
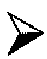 *H. pylori* mainly resides in the gastric mucosa or at the interface with the mucus layer and poor diffusion of antibiotics to mucus layer or insufficient release of antibiotics at mucus.
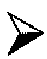 Unstability of antibiotics in different pH especially gastric conditions
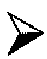 Rapid degradation of many antibiotics in gastric acid
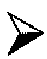 Gastric emptying and inadequate residence time of drugs in stomach
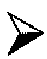 Catalase activity of *H.pylori*
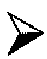 Self-protection of *H.pylori* against physical and chemical stresses via transforming coccoid form by changing morphological, metabolism and reproduction characteristic.
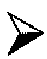 Antibiotic resistance of *H. pylori*
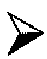 Allergy of patients against antibiotics.

The related invention presents an alternative solution which increases success of the therapy and provides controlled release in gastrointestinal system to current products. The advantage of the invention is not only eradication of *H.pylori* by controlled release but also preventing the adhesion/colonization of *H.pylori* to gastric mucosa and providing wound healing. This invention is chitosan nanocomposite controlled release system which comprises combination of chitosan, nanoclay and specified amount of essential oil which has antimicrobial property. The cinnamon, thyme, clove, lemongrass and lemon oil have antimicrobial activity against *H.pylori.*

Chitosan microspheres containing specific essential oil at specified amount of MIC value (Table 2) are obtained by addition of the oil following the incorporation of organic modified montmorillonite clay. Final product is obtained by spray drying method in microsphere form after emulsification process of essential oil, chitosan and nanoclay. The particle size of final product is smaller than 5 µm.

**Table 2. Minimum inhibition concentration (MIC) of essential oils against H.pylori**

| **Essential oil** | **MIC value (µg/mL)** |
|---|---|
| Cinnamon bark oil | 8 |
| Lemongrass | 62 |
| Thyme | 62 |
| Clove | 125 |
| Lemon | 500 |

Approximately 80% of cinnamon oil released into simulated gastric fluid (SGF) from native chitosan (pure chitosan) microspheres. There was no significant change in oil concentration after 1 hour. Related substances are released into the medium in a very short time due to rapid swelling and degradation of chitosan based microspheres in SGF. It is important to acquire the long-term stability in stomach by adhesion to mucus layer (3) and controlled release profile in *H.pylori* (4) eradication. Long-term stability in stomach by adhesion to mucus layer and controlled release profile are important in *H.pylori* eradication. Thus innovative solutions which improve stability and release profile of chitosan based drug delivery systems in stomach are needed. To overcome these disadvantages, layered silicates introduce into chitosan matrix to obtain nanocomposite structure. Controlled release profile was obtained by incorporating montmorillonite clay into the structure. By this way, encapsulation efficiency of essential oil (cinnamon, thyme, clove, lemon and lemongrass oil) increases and sustained release is provided.

The related invention is essential oil loaded chitosan nanocomposite controlled release system. Nanoclay within the chitosan naocomposite controlled release system consists of layered silicate structure (1). Essential oils are encapsulated in different polymeric materials. Exfoliated structure of layered silicates in polymer matrix provides tortuous pathway (2) for molecules which diffuse through the nanocomposite and release in a controlled manner.

Chitosan exhibits bioadhesive and permeability properties which have a significant effect on drug release. Chitosan enhances the passage of active agent through the mucus layer with nanocomposite structure (5). Chitosan also is known to have the ability to transiently open epithelial tight junctions, thus facilitating the transport of active agent molecules through well-organized epithelial cells (6) by acting as an absorption promoter. The mechanism underlying chitosan effect relies on the interaction of its protonated amino groups with cell membranes. Aforesaid chitosan here, is high molecular weight and has positive surface charge (zeta potential) as presented in Table 3. Chitosan has mucoadhesive property due to the amine groups in the polymer chain which forms hydrogen bonds with glycoproteins in mucus and electrostatic interaction of positively charged groups with negatively charged mucus layer and thus increases the residence time of drug. It also inhibits adhesion of *H.pylori* by binding the gastric mucosa. Aforementioned nanocomposite release system contains nanoclay in the form of layered silicate. Although there are several studies on the use and/or encapsulation of essential oils in different fields, this invention presents a controlled release of essential oil from nanocomposite system which is more effective eradication treatment on *H.pylori.* It is designed the produced nanocomposite release system is desired in the form of sphere. The size of spheres should be below 5 µm for cellular uptake of bacteria and epithelial cells (6).

**Table 3. Surface charge of oil loaded chitosan microspheres**

| **Formulation** | | **Zeta Potential (mV)** |
|---|---|---|
| Nanoclay (%) | Polymer (%) | |
| 0 | 1 | 34 |
| 1 | 1 | 36 |
| 3 | 1 | 33 |
| 5' | 1 | 31 |

This invention is explained in more detail based on the following example and experiment and not intended to limit the subject matter of the present invention.

### Example 1:

1 wt% of chitosan solution was prepared by dissolving of chitosan in 2 % acetic acid. 3% (w/w) MMT clay was dissolved in 2(v/v)% acetic acid, too. Chitosan and MMT solution were mixed together under magnetic stirring and then sonicated. 1% (v/v) of cinnamon oil was added into final solution and the emulsion was sonicated for 30 min. After the sonication, emulsion was dried by spray drying at 190 °C. The final product is microsphere which has mucoadhesive property and antimicrobial activity against *H.pylori.*

### The Industrial Application of This Invention

The present invention will be in the form of chitosan-nanoclay nanocomposite controlled release systems which provide local treatment on H. pylori in the stomach. The application areas could be though as food supplement of pharmaceutical coating excipient. Thefore, this invention which adressess the abovementioned objectives is suitable for usage in pharmaceutical sector and applicable to industry.

## Claims

1. Chitosan-nanoclay nanocomposite controlled release system comprising essential oil and nanoclay, for use in the local treatment of infection caused by *Helicobacter pylori.*

2. Controlled release system for use according to claim 1 where the essential oil is selected from cinnamon bark, thyme, lemon, lemongrass and clove oils.

3. Controlled release system for use according to claim 2 comprising therapeutically effective amount of essential oil at least equal to minimum inhibition concentration (MIC) against H.pylori.

4. Controlled release system for use according to claim 2 comprising therapeutically effective amount of cinnamon bark oil at least equal to minimum inhibition concentration (MIC) against H.pylori.

5. Controlled release system for use according to claim 1 where nanoclay has layered silicate structure.

6. Controlled release system for use according to claim 1 where chitosan is high molecular weight and has positive zeta potential (surface charge) in gastric conditions.

7. Controlled release system for use according to claim 1 where chitosan-nanoclay nanocomposite controlled release system is in the form of spheres.

8. Controlled release system for use according to claim 7 where sphere has particle size below 5 µm.

## Patentansprüche

1. Chitosan-Nanoton-Nanokomposit-System zur kontrollierten Freisetzung, bestehend aus ätherischem Öl und Nanoton, zur lokalen Behandlung von Infektionen durch *Helicobacter pylori.*

2. System zur kontrollierten Freisetzung zur Verwendung nach Anspruch 1, wobei das ätherische Öl aus Zimtrinde, Thymian, Zitrone, Zitronengras und Nelkenöl ausgewählt wird.

3. System zur kontrollierten Freisetzung zur Verwendung nach Anspruch 2, bestehend aus einer therapeutisch wirksamen Menge an ätherischem Öl, welche mindestens der minimalen Hemmkonzentration (MHK) gegen *H. pylori* entspricht.

4. System zur kontrollierten Freisetzung zur Verwendung nach Anspruch 2, bestehend aus einer therapeutisch wirksamen Menge an Zimtrindenöl, welche mindestens der minimalen Hemmkonzentration (MHK) gegen *H. pylori* entspricht.

5. System zur kontrollierten Freisetzung zur Verwendung nach Anspruch 1, wobei der Nanoton eine Schichtsilikatstruktur aufweist.

6. System zur kontrollierten Freisetzung zur Verwendung nach Anspruch 1, wobei Chitosan ein hohes Molekulargewicht hat und ein positives Zeta-Potential (Oberflächenladung) unter Magenbedingungen aufweist.

7. System zur kontrollierten Freisetzung zur Verwendung nach Anspruch 1, wobei das Chitosan-Nanoton-Nanokomposit-System zur kontrollierten Freisetzung in Form von Kügelchen vorliegt.

8. System zur kontrollierten Freisetzung zur Verwendung nach Anspruch 7, wobei die Kügelchen eine Partikelgröße von unter 5 µm aufweisen.

## Revendications

1. Système à libération contrôlée nano-composite de nano-argile de chitosane comprenant de l'huile essentielle et du nano-argile pour traitement local de l'infection causée par Helicobacter pylori.

2. Système à libération contrôlée pour utilisation selon la revendication 1, dans lequel l'huile essentielle est choisie parmi les huiles d'écorce de cannelle, de thym, de citron, de citronnelle et de girofle.

3. Système à libération contrôlée pour utilisation selon la revendication 2 comprenant une quantité efficace thérapeutiquement d'huile essentielle au moins égale à la concentration minimale d'inhibition (MIC) contre H.pylori.

4. Système à libération contrôlée pour utilisation selon la revendication 2 comprenant une quantité efficace thérapeutiquement d'huile d'écorce de cannelle au moins égale à la concentration minimale d'inhibition (MIC) contre H.pylori.

5. Système à libération contrôlée pour utilisation selon la revendication 1, dans lequel le nano-argile possède une structure de silicate en couches.

6. Système à libération contrôlée pour utilisation selon la revendication 1, dans lequel le chitosane a un poids moléculaire élevé et un potentiel zêta positif (charge de surface) dans des conditions gastriques.

7. Système à libération contrôlée pour utilisation selon la revendication 1, dans lequel le système à libération contrôlée nano-composite de chitosane-nano-argile est en forme de sphères.

8. Système à libération contrôlée pour utilisation selon la revendication 7, dans lequel la sphère a une taille de particules qui est inférieure à 5 µm.
